# EUROPÄISCHE PATENTSCHRIFT

(11) **EP 3 027 168 B1**
(45) Veröffentlichungstag und Bekanntmachung des Hinweises auf die Patenterteilung: **05.07.2017**
(21) Anmeldenummer: 14733981.6
(22) Anmeldetag: 15.05.2014
(51) Int. Cl.: A61K 8/25, A61K 8/29, A61K 8/37, A61Q 19/00, A61K 8/92, A61K 8/06, A61Q 17/04, A61K 8/891, A61K 8/02

(54) **WASSERFREIE ZUSAMMENSETZUNG MIT LEICHTER TEXTUR ZUM AUFTRAG AUF DIE HAUT**
WATER-FREE COMPOSITIONS OF A SMOOTH TEXTURE FOR APPLICATION ONTO THE SKIN
COMPOSITIONS SANS EAU AVEC UNE TEXTURE RÉDUITE POUR L'APPLICATION SUR LA PEAU

(30) Priorität: 30.07.2013 DE 102013214843
(43) Veröffentlichungstag der Anmeldung: 08.06.2016
(73) Patentinhaber: Henkel AG & Co. KGaA, 40589 Düsseldorf (DE)
(72) Erfinder: STRUWE, Alexandra, 47877 Willich (DE)
(86) Internationale Anmeldenummer: PCT/DE2014/200218
(87) Internationale Veröffentlichungsnummer: WO 2015/014351

(56) Entgegenhaltungen:
- WO-A1-2005/027867
- CA-A1- 2 687 641
- US-A1- 2007 166 253

## Beschreibung

Die vorliegende Erfindung betrifft Hautkosmetika, insbesondere wasserfreie Zusammensetzungen mit spezieller Dichte, die flüssiges Öl und partikelförmig dispergierten Feststoff enthalten. WO2005027867 offenbart eine Zusammensetzung bestehend aus 58,5 % Jojobaöl, 12,0 % Bienenwachs, Lecithin, Talkum, Polyphenolextrakt des grünen Tees, Sorbinsäure und Ascorbylpalmitat wie Lavendelöl und Teebaumöl. In US2007166253 ist eine Lippenstiftformulierung dargelegt, die eine Reihe von Ölkomponenten enthält, nämlich 8.06 % Diisostearylmalat, 12.6 % Dipentaerythritylhexahydroxystearat/ hexastearat/hexarosinat, 0,6 % Grapefruitkernöl, ferner u.a. 9 % Vaseline als Wachs, Titandioxid, Stearinsäure, Aluminiumhydroxid, 2.0 % Glimmer und Isopropyltitaniumtriisostearat, 3,0 % Glimmer und Titandioxid, 5,0 % Kieselsäure und 1,5 % Pfefferminzöl. CA26876641 bezieht sich auf einen Lippenstift, der organisches Kokosnussöl und Bienenwachs enthält. Kosmetika mit partikelförmig dispergierten Feststoffen sind dem Fachmann bekannt. Diese werden beispielsweise in der Hautkosmetik als UV-Schutzkosmetikum mit Titandioxid, als Antitranspirans mit partikelförmigen Aluminiumsalzen oder zur dekorativen Kosmetik als Schminke mit partikelförmigen Farbpigmenten in Form von Puder oder Lippenstiften eingesetzt. Auch der Einsatz von Feststoffpartikeln zur Erzeugung optischer Effekte wie Perlglanz ist bekannt.

Problematisch bei der Formulierung von Kosmetika mit partikelförmigen Feststoffen ist einerseits die stabile Einarbeitung der Feststoffpartikel in ein flüssiges Medium. Die Partikel sollen lagerstabil eingearbeitet sein und nicht sedimentieren. Bei Kosmetika mit hohem Ölgehalt muss ferner das Phänomen der Synärese verhindert werden. Bei der Synärese wird die Zusammensetzung, beispielsweise bei Ausübung von Druck, instabil. Dabei scheidet sich ein flüssiger Bestandteil der Zusammensetzung ab. Eine Sedimentierung der eingearbeiteten Feststoffpartikel muss während der Synärese nicht zwingend erfolgen.

Kosmetika mit insbesondere hohem Anteil partikelförmiger Feststoffe werden vom Verbraucher oftmals als unpraktisch in der Handhabung und unangenehm nach der Applikation empfunden. Sie lassen sich oftmals schwer auf dem Substrat, z.B. der Haut, verteilen und hinterlassen auf dem Substrat ein unangenehmes Gefühl. Bei Kosmetika mit hohem Ölgehalt wird oft ein unangenehmes schmieriges Gefühl wahrgenommen. Der Verbraucher spürt jeweils die Beschichtung mit dem Kosmetikum nachhaltig. Er wünscht sich trotz der Gegenwart der Pigmente und ggf. der Öle auf der Haut ein samtiges, leichtes Hautgefühl.

Aufgabe der vorliegenden Anmeldung war es daher, ein lagerstabiles Kosmetikum, bereitzustellen, das als Feststoff dispergierte Partikel enthält und sich leicht auf einem Substrat, insbesondere der Haut, verteilen lässt. Ferner soll das besagte Kosmetikum ein samtiges und leichtes Hautgefühl vermitteln.

Es wurde gefunden, dass die gestellte Aufgabe durch eine viskose öl- und feststoffpartikelhaltige Zusammensetzung gelöst wird, die eine spezielle Dichte und somit eine mousseartige Textur aufweist.

Ein erster Erfindungsgegenstand sind daher kosmetische Mittel, enthaltend jeweils bezogen auf das Gesamtgewicht des Mittels
- 0 bis 3 Gew.-%, insbesondere 0 bis 2 Gew.%, freies Wasser,
- ein oder mehrere flüssige Öle in einer Gesamtmenge von 40 bis 80 Gew.-%,
- mindestens ein Wachs,
- partikelförmig dispergierten Feststoff,
- gegebenenfalls weitere Inhaltsstoffe, wie im Anspruch 1 dargestellt. wobei
- das kosmetische Mittel eine Viskosität von 300000 bis 800000 mPas, insbesondere von 400000 bis 600000 mPas (jeweils gemessen bei 20°C, Brookfield, 4 Umdrehungen pro Minute, Spindel TE) besitzt,
- das kosmetische Mittel bei 20°C eine Dichte von 0,95 bis 1,10 g/cm³ hat.

Der Begriff "freies Wasser" wird erfindungsgemäß so verstanden, dass der Gehalt an Kristallwasser, Hydratationswasser oder ähnlich molekular gebundenem Wasser, der in den eingesetzten Bestandteilen, insbesondere in den partikelförmig dispergierten Feststoffen, enthalten sein kann, im Sinne der vorliegenden Anmeldung kein freies Wasser darstellt.

Alle Angaben über die Aggregatzustände von Stoffen (fest, flüssig, gasförmig) in dieser Anmeldung beziehen sich auf Normalbedingungen. "Normalbedingungen" sind im Sinne der vorliegenden Anmeldung eine Temperatur von 20 °C und ein Druck von 1013,25 mbar. Schmelzpunktangaben beziehen sich ebenfalls auf einen Druck von 1013,25 mbar.

Die erfindungsgemäße Zusammensetzung enthält weiterhin zwingend mindestens ein oder mehrere flüssige Öle in besagter Gesamtmenge. Unter einem Öl ist erfindungsgemäß ein flüssiger Stoff zu verstehen, der bei Normalbedingungen in bidestilliertem Wasser zu weniger als 1 Gew.-% mischbar ist.

In einer bevorzugten Ausführungsform wird das flüssige Öl ausgewählt aus mindestens einer Verbindung der Gruppe, die gebildet wird aus flüssigen Silikonen; Esterölen; Trifettsäureester von gesättigten und/oder ungesättigten, linearen und/oder verzweigten C₆- bis C₂₂-Fettsäuren mit Glycerin; pflanzlichen Ölen; flüssigen Paraffinölen; Isoparaffinölen; flüssigen synthetischen Kohlenwasserstoffen; flüssigen Di-n-alkylethern; Dicarbonsäureestern; symmetrischen, unsymmetrischen oder cyclischen Estern der Kohlensäure.

Es ist erfindungsgemäß bevorzugt, wenn das erfindungsgemäße Mittel bezogen auf das Gewicht der Zusammensetzung flüssiges Öl in einer Gesamtmenge insbesondere von 50 bis 75 Gew.-%, insbesondere von 60 bis 70 Gew.-%, in der erfindungsgemäßen Zusammensetzung enthalten sind. Bevorzugt enthält die erfindungsgemäße Zusammensetzung als flüssiges Öl mindestens ein Silikonöl.

Es ist erfindungsgemäß bevorzugt, wenn das erfindungsgemäße Mittel bezogen auf das Gewicht der Zusammensetzung Silikonöl in einer Gesamtmenge von 30 bis 70 Gew.-%, insbesondere von 40 bis 65 Gew.-%, in der erfindungsgemäßen Zusammensetzung enthält.

Wiederum bevorzugt werden die Silikonöle ausgewählt, aus mindestens einer Verbindung aus der Gruppe, die gebildet wird aus:
(i) Polyalkylsiloxanen, Polyarylsiloxanen, Polyalkylarylsiloxanen, die flüchtig oder nicht flüchtig, geradkettig, verzweigt oder cyclisch, vernetzt oder nicht vernetzt sind;
(ii) Polysiloxanen, die in ihrer allgemeinen Struktur eine oder mehrere organofunktionelle Gruppen enthalten, die ausgewählt sind unter (per)fluorierten Gruppen;
(iii) oder deren Gemischen.

Ganz besonders bevorzugte kosmetische Mittel sind dadurch gekennzeichnet, dass sie als Öl mindestens ein Silikon der Formel (Si-1)

(CH₃)₃Si-[O-Si(CH₃)₂]ₓ-O-Si(CH₃)₃ (Si-1),

enthalten, in der x für eine Zahl von 0 bis 200, vorzugsweise von 0 bis 100, weiter bevorzugt von 0 bis 20, steht.

Die erfindungsgemäß bevorzugten kosmetischen Mittel enthalten als Öl mindestens ein Silikon der vorstehenden Formel (Si-1). Diese Silikone werden nach der INCl-Nomenklatur als Dimethicone bezeichnet.

Erfindungsgemäß ebenfalls bevorzugte Silikonöle sind ausgewählt aus Silikonen der Formel (Si-2) wobei x ausgewählt ist aus ganzen Zahlen von 1 - 20, bevorzugt 1 - 3.

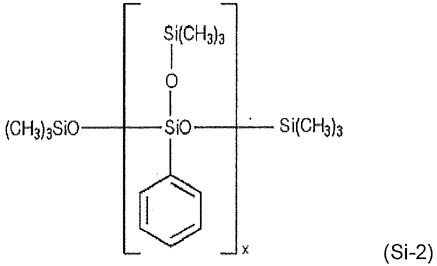

Ein bevorzugtes Silikonöl der Formel (Si-2) ist erhältlich unter der INCI-Bezeichnung Phenyl Trimethicone.

Selbstverständlich können auch Mischungen der o.g. Silikone der Formel (Si-1) und (Si-2) als Silikonöl in den bevorzugten erfindungsgemäßen Mitteln enthalten sein.

Bevorzugte erfindungsgemäß einsetzbare Silikonöle, insbesondere gemäß Formel (Si-1), weisen bei 25°C eine kinematische Viskosität von 1 bis 200 mm²s⁻¹, besonders bevorzugt von 5 bis 100 mm²s⁻¹. Solche Silikonöle sind beispielsweise als Dimethicone unter dem Handelsnamen Xiameter PMX 200 Sil Fluid 50 CS (früher: Dow Corning 200 fluid 50 cSt) im Handel erhältlich.

Im Rahmen einer weiteren Ausführungsform der Erfindung sind solche erfindungsgemäßen kosmetischen Mittel bevorzugt, die als flüssiges Öl mindestens einen Ester der Formel (I) enthalten 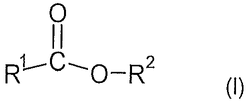 worin R¹ und R² unabhängig voneinander für eine lineare oder verzweigte Kohlenwasserstoffgruppe mit 3 bis 22 Kohlenstoffatomen stehen.

Es ist besonders bevorzugt, wenn gemäß Formel (I) R¹ eine lineare oder verzweigte Kohlenwasserstoffgruppe mit 3 bis 22 Kohlenstoffatomen und R² eine verzweigte Kohlenwasserstoffgruppe mit 3 bis 22 Kohlenstoffatomen ist.

Bevorzugte flüssige Öle werden ausgewählt aus Estern gemäß Formel (I) von C₆ bis C₂₂ - Fettsäuren (R¹ = linearer oder verzweigter C₅-C₂₁ Kohlenwasserstoff) mit C₃ bis C₂₂ - Fettalkoholen (R² = linearer oder verzweigter C₃ bis C₂₂ Kohlenwasserstoff).

Bevorzugte Beispiele für eingesetzte Fettsäurenanteile in den Estern der Formel (I) sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren Mischungen, wie z.B. die technischen Mischungen, die z.B. bei der Druckspaltung von natürlichen Fetten und Ölen, bei der Oxidation von Aldehyden aus der Roelen'schen Oxosynthese oder der Dimerisierung von ungesättigten Fettsäuren anfallen. Bevorzugte Beispiele für die Fettalkoholanteile in den Estern der Formel (I) sind Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren Mischungen, wie z.B. die technischen Mischungen, die bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen.

Erfindungsgemäß besonders bevorzugt, wird die Verbindung gemäß Formel (I) ausgewählt unter 2-Ethylhexylpalmitat, 2-Ethylhexylstearat, 2-Ethylhexylcocoat, 2-Ethylhexyllaurat, 2-Ethylhexylisostearat, Hexyldecylstearat, Hexyldecyllaurat, Isodecylneopentanoat, Isononylisononanoat, Isopropylmyristat, Isopropylpalmitat, Isopropylstearat, Isopropylisostearat, Isopropyloleat, Isooctylstearat, Isononansäure-C₁₆₋₁₈-alkylester, Isononylstearat, Isocetylstearat, Isononylisononanoat, Isotridecylisononanoat, Cetearylisononanoat, 2-Octyldodecylpalmitat, 2-Octyldodecylmyristat, 2-Octyldodecyllaurat, 2-Octyldodecylstearat, Butyloctansäure-2-butyloctanoat, Kokosfettalkoholcaprinat/-caprylat, n-Butylstearat, n-Hexyllaurat, n-Decyloleat, Cetyloleat, Oleyloleat, Oleylerucat, Erucyloleat, Erucylerucat, Gemischen aus zwei oder mehreren der vorgenannten Verbindungen.

Es ist erfindungsgemäß besonders bevorzugt, die jeweiligen Kohlenwasserstoffreste der Ester der Formel (I), insbesondere im Rahmen der bevorzugten Ausführungsformen besagter Ester *(vide supra*), aus gesättigten Kohlenwasserstoffen auszuwählen.

Die erfindungsgemäßen Mittel enthalten die Verbindungen der Formel (I) bevorzugt in einer Menge von 1 bis 60 Gew.-%, insbesondere von 5 bis 30 Gew.-%, jeweils bezogen auf das Gesamtgewicht des Mittels.

Im Rahmen dieser Ausführungsform der Erfindung sind wiederum solche erfindungsgemäßen kosmetischen Mittel bevorzugt, die als flüssiges Öl zumindest enthalten
- mindestens einen Ester der Formel (I) 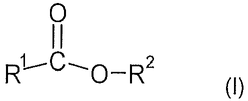 worin R¹ und R² unabhängig voneinander für eine lineare oder verzweigte Kohlenwasserstoffgruppe mit 3 bis 20 Kohlenstoffatomen stehen, und
- mindestens ein Silikonöl.
   Im Rahmen dieser Kombination sind wiederum die bevorzugten Ausführungsformen der Verbindungen der Formel (I) und der Silikonöle als besonders bevorzugte Komponenten einsetzbar. Im Rahmen obiger Ausführungsform der Erfindung sind solche erfindungsgemäßen kosmetischen Mittel besonders bevorzugt, die als flüssiges Öl zumindest enthalten,
- einen oder mehrere Ester der Formel (I) 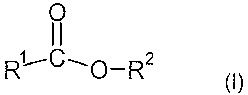 worin R¹ und R² unabhängig voneinander für eine lineare oder verzweigte Kohlenwasserstoffgruppe mit 3 bis 20 Kohlenstoffatomen stehen, in einer Gesamtmenge von 1 bis 20, insbesondere von 5 bis 15 Gew.-%,
- Silikonöl in einer Gesamtmenge von 30 bis 60 Gew.-%, insbesondere von 40 bis 60 Gew.-%, Im Rahmen dieser Kombination sind wiederum die bevorzugten Ausführungsformen der Verbindungen der Formel (I) und der Silikonöle als besonders bevorzugte Komponenten einsetzbar.

Als flüssiges Öl erfindungsgemäß geeignet sind auch Triglyceridöle wie die flüssigen Anteile des Rindertalgs sowie synthetische Triglyceridöle. Bevorzugte Triglyceridöle werden ausgewählt unter den Triglyceriden von linearen oder verzweigten, gesättigten oder ungesättigten, gegebenenfalls hydroxylierten C₈₋₃₀-Fettsäuren. Besonders geeignet kann die Verwendung natürlicher Öle, z.B. Sojaöl, Baumwollsaatöl, Sonnenblumenöl, Palmöl, Palmkernöl, Leinöl, Mandelöl, Rizinusöl, Maisöl, Rapsöl, Olivenöl, Sesamöl, Distelöl, Weizenkeimöl, Pfirsichkernöl und die flüssigen Anteile des Kokosöls, Glyceryltriölsäureester (Triolein), Capric/Caprylic Triglyceride, Glyceryltriisostearin, Glyceryltriisopalmitat und Mischungen aus zweien oder mehreren der vorgenannten Verbindungen. Bevorzugte pflanzliche Öle werden ausgewählt aus mindestens einem Vertreter der Gruppe, die gebildet wird aus Amaranthöl, Sonnenblumenöl, Olivenöl, Sojaöl, Rapsöl, Mandelöl, Jojobaöl, Orangenöl, Aprikosenkernöl, Macadamianussöl, Weizenkeimöl, Pfirsichkernöl und die flüssigen Anteile des Kokosöls.

Bevorzugte Di-n-alkylether werden ausgewählt aus Di-n-alkylethern mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether, Di-n-dodecylether, n-Hexyl-n-octylether, n-Octyl-n-decylether, n-Decyl-n-undecylether, n-Undecyl-n-dodecylether und n-Hexyl-n-Undecylether sowie Di-tert-butylether, Di-iso-pentylether, Di-3-ethyldecylether, tert.-Butyl-n-octylether, iso-Pentyl-n-octylether und 2-Methyl-pentyl-n-octylether. Die als Handelsprodukte erhältlichen Verbindungen 1,3-Di-(2-ethyl-hexyl)-cyclohexan (Cetiol^{®} S) und Di-n-octylether (Cetiol^{®} OE) können bevorzugt sein.

Bevorzugte Dicarbonsäureester werden ausgewählt aus mindestens einer Verbindung der Gruppe, die gebildet wird, aus Di-n-butyladipat, Di-(2-ethylhexyl)-adipat, Di-(2-ethylhexyl)-succinat und Diisotridecylacelaat sowie Diolestern wie Ethylenglykol-dioleat, Ethylenglykol-di-isotridecanoat, Propylenglykol-di(2-ethylhexanoat), Propylenglykol-di-isostearat, Propylenglykol-di-pelargonat, Butandiol-di-isostearat, Neopentylglykoldicaprylat.

Natürliche und synthetische Kohlenwasserstoffe, wie beispielsweise Paraffinöle, C₁₈-C₃₀-Isoparaffine, insbesondere Isoeicosan, Polyisobutene oder Polydecene, die beispielsweise unter der Bezeichnung Emery^{®} 3004, 3006, 3010 oder unter der Bezeichnung Ethylflo^{®} von Albemarle oder Nexbase^{®} 2004G von Nestle erhältlich sind, sowie 1,3-Di-(2-ethylhexyl)-cyclohexan (erhältlich z. B. unter dem Handelsnamen Cetiol^{®}S von Cognis) gehören ebenfalls zu den erfindungsgemäß bevorzugt einsetzbaren flüssigen Ölen.

Weitere erfindungsgemäß bevorzugte flüssige Öle sind ausgewählt aus den Benzoesäureestern von linearen oder verzweigten C₈₋₂₂-Alkanolen. Besonders bevorzugt sind Benzoesäure-C12-C15-alkylester, z. B. erhältlich als Handelsprodukt Finsolv^{®} TN, Benzoesäureisostearylester, z. B. erhältlich als Handelsprodukt Finsolv^{®} SB, Ethylhexylbenzoat, z. B. erhältlich als Handelsprodukt Finsolv^{®} EB, und Benzoesäureoctyldocecylester, z. B. erhältlich als Handelsprodukt Finsolv^{®} BOD.

Weitere erfindungsgemäß bevorzugte flüssige Öle sind ausgewählt aus den Dicarbonsäureestern von linearen oder verzweigten C₂-C₁₀-Alkanolen, insbesondere Diisopropyladipat, Di-n-butyladipat, Di-(2-ethylhexyl)adipat, Dioctyladipat, Diethyl-/Di-n-butyl/ Dioctylsebacat, Diisopropylsebacat, Dioctylmalat, Dioctylmaleat, Dicaprylylmaleat, Diisooctylsuccinat, Di-2-ethylhexylsuccinat und Di-(2-hexyldecyl)-succinat.

Weitere erfindungsgemäß bevorzugte flüssige Öle sind ausgewählt aus den symmetrischen, unsymmetrischen oder cyclischen Estern der Kohlensäure mit Alkanolen, z. B. Glycerincarbonat, Dicaprylylcarbonat (Cetiol^{®} CC) oder die Ester gemäß der Lehre der DE 19756454 A1.

Weiterhin enthalten die erfindungsgemäßen Mittel zwingend mindestens ein Wachs. Unter Wachsen werden im Allgemeinen Feststoffe verstanden, die Rekationsprodukte von Carbonsäuren und Alkoholen sind. Diese Reaktionsprodukte sind als Wachs fest bis brüchig hart, bis 20°C knetbar und schmelzen bei 30°C bis 90°C.

Erfindungsgemäße Wachse weisen bevorzugt einen Schmelzpunkt in einem Bereich von 30°C bis 60°C, besonders bevorzugt in einem Bereich von 30°C bis 50°C, ganz besonders bevorzugt in einem Bereich von 30°C bis 40°C, auf.

Es ist erfindungsgemäß bevorzugt, wenn mindestens die Hälfte der Menge des enthaltenen Wachses (besonders bevorzugt mindestens 75% der Menge des enthaltenen Wachses) im flüssigen Öl des erfindungsgemäßen kosmetischen Mittels gelöst vorliegt. Wenn im Zusammenhang mit dieser Ausführungsform mindestens ein Ester der Formel (I) *(vide supra*) in der flüssigen Ölkomponente enthalten ist, so ist es im Rahmen des Herstellprozesses erfindungsgemäß bevorzugt, Wachs in einer Menge mindestens eines Esters der Formel (I) *(vide supra*) zu lösen und dann in dieser gelösten Form in das erfindungsgemäße kosmetische Mittel einzuarbeiten.

Erfindungsgemäß ganz besonders bevorzugt, enthält das erfindungsgemäße Mittel mindestens ein Wachs, ausgewählt aus mindestens einem Wachs der Gruppe Wachse mit der INCI-Bezeichnung Cocoglycerides, Cetylpalmitat, Myristylmyristat.

Die erfindungsgemäßen Mittel enthalten bevorzugt Wachs in einer Gesamtmenge von 0,05 bis 8,0 Gew.-%, besonders bevorzugt von 0,1 bis 5,0 Gew.-%, ganz besonders bevorzugt von 0,5 bis 3,0 Gew.-%, jeweils bezogen auf das Gesamtgewicht des Mittels.

Die erfindungsgemäßen kosmetischen Mittel enthalten zwingend partikelförmig dispergierten Feststoff. Im Sinne der Erfindung sind Partikel als Korn vorliegende Teilchen (vgl. DIN 66160: 1992-09) von Festkörpern. In dem erfindungsgemäßen Mittel liegen folglich als Korn vorliegende Teilchen von Festkörpern als solche dispergiert vor.

Die Partikel weisen bevorzugt einen mittleren Teilchendurchmesser (Volumenmittel) von 0.01 bis 3,0 µm, insbesondere von 0,05 bis 1,0 µm, ganz besonders bevorzugt von 0,1 bis 0,5 µm.

Es sind erfindungsgemäß solche kosmetischen Mittel bevorzugt, die partikelförmig dispergierten Feststoff in einer Gesamtmenge von 10 bis 30 Gew.-% enthalten.

Als partikelförmig dispergierter Feststoff ist in bevorzugten erfindungsgemäßen kosmetischen Mitteln mindestens ein anorganischer Feststoff, insbesondere mindestens ein Metalloxid, enthalten. Die Metalloxide werden wiederum bevorzugt ausgewählt aus Aluminaten, Aluminiumsilikaten, Titandioxid, Zinkdioxid, Eisenoxiden, Zinndioxid, Perlglanzpigmenten sowie Gemischen aus zwei oder mehreren der vorgenannten Metalloxide.

Häufig verwendete Perlglanzpigmente sind Perlmutt (aus vermahlenen Muschelschalen), monokristalline Perlglanzpigmente wie z.B. Bismutoxychlorid, sowie Perlglanzpigmente auf Basis von Glimmer, Glimmer/Metalloxid oder Titandioxid/Metalloxid. Letztgenannte Perlglanzmischpigmente werden mit einer vom Partikelmaterial des Kerns verschiedenen Metalloxidbeschichtung versehen. Durch den Einsatz der Perlglanzpigmente werden Farbeffekte und/oder Glanz in den erfindungsgemäßen Mitteln oder auf dem damit behandelten Substrat erzielt.

Besonders bevorzugte Aluminate werden ausgewählt unter aktivem Aluminiumoxid, alpha-Aluminiumoxid, beta-Aluminiumoxid, gamma-Aluminiumoxid sowie deren Gemischen.

Besonders bevorzugte Aluminiumsilikate (auch Alumosilikate genannt) werden ausgewählt unter Phyllosilikaten, Tectosilikaten.

Bevorzugt geeignete Phyllosilikate werden ausgewählt unter Kaolinen (hier insbesondere unter Kaolinit, Dickit, Hallosit sowie Nakrit), Serpentin, Talk, Pyrophyllit, Montmorillonit, Quarz, Bentonit, Glimmer (hier insbesondere unter Illit, Muscovit, Paragonit, Phlogopit, Biotit, Lepidolith, Margarit, Smektit (hier insbesondere unter Montmorrilionit, Saponit, Nontronit, Hectorit)).

Bevorzugt geeignete Tectosilikate werden ausgewählt unter Feldspatmineralien (insbesondere Albit, Orthoklas, Anorthit, Leucit, Sodalith, Hauyn, Labradorit, Lasurit, Nosean, Nephelin).

Bevorzugte Titandioxide sind solche, die unter dem Handelsnamen Kronos von der Firma Kronos vertrieben werden, insbesondere Kronos 1171.

Bevorzugte Eisendioxide sind Fe₂O₃, beispielsweise mit der INCI Cl 77491, insbesondere als Unipur Red LC 281 EM^{®} von der Fa. Sensient im Handel, Fe₂O₃ · n H₂O, beispielsweise mit der INCI Cl 77491, insbesondere als Unipur Red LC 281 EM^{®} von der Fa. Sensient im Handel, FeO · Fe₂O₃, beispielsweise mit der INCI Cl 77499, insbesondere als Unipur Black LC 989 EM^{®} von der Fa. Sensient im Handel.

Erfindungsgemäß bevorzugte kosmetische Mittel sind dadurch gekennzeichnet, dass sie (gemeinsam mit vorgenannten anorganischen Feststoffen oder ohne die Gegenwart von vorgenannten anorganischen Feststoffen als partikelförmig dispergierten Feststoff) als partikelförmig dispergierten Feststoff mindestens eine Stärke enthält.

Stärke ist ein Reservekohlenhydrat, das von vielen Pflanzen in Form von üblicherweise 1 bis 200 µm großen Stärkekörnern (Granula) in verschiedenen Pflanzenteilen gespeichert wird, z.B. in Knollen oder Wurzeln, Getreidesamen, Früchten sowie im Mark. Stärke gehört zu der Familie der Homoglycane und ist ein Polykondensationsprodukt von D-Glucose. Dabei besteht Stärke aus drei strukturell verschiedenen Polymeren der d-Glucopyranose, nämlich der Amylose, dem Amylopektin und einer sogenannten Zwischenfraktion. Höhere Pflanzen enthalten 0 bis 45 Gew.% Amylose bezogen auf die Trockensubstanz. Die Zwischenfraktion, die auch als anormales Amylopektin bezeichnet wird, steht strukturell zwischen der Amylose und dem Amylopektin. Die im Rahmen dieser Anmeldung definierten Mengenangaben für Amylopektin umfassen die Zwischenfraktion. Amylose besteht aus überwiegend linear α-1,4-glycosidisch verknüpfter d-Glucose, Mr 50000-150000. Die resultierenden Ketten bilden in der Stärke Doppelhelices. Amylopektin enthält neben den für Amylose beschriebenen α-1,4-Verknüpfungen auch in einer Menge von 4 bis 6% α-1,6-Bindungen als Verzweigungsstellen. Der durchschnittliche Abstand zwischen den Verzweigungsstellen beträgt etwa 12 bis 17 Glucose-Einheiten. Die Molmasse von 107 bis 7 · 108 entspricht ca. 105 Glucose-Einheiten, womit Amylopektin zu den größten Biopolymeren gehört. Besagte Verzweigungen sind derart über das Molekül verteilt, daß sich eine Büschelstruktur mit relativ kurzen Seitenketten entwickelt. Zwei dieser Seitenketten bilden jeweils eine Doppelhelix. Durch die vielen Verzweigungsstellen ist Amylopektin in Wasser relativ gut löslich.

Eine erfindungsgemäß bevorzugt verwendbare Stärke wird ausgewählt unter mindestens einem Polykondensationsprodukt von D-Glucose erhalten aus Stärke von Kartoffeln, Mais, Reis, Erbsen, Eicheln, Kastanien, Gerste, Weizen, Bananen, Sago, Hirse, Sorghum, Hafer, Gerste, Roggen, Bohnen, Batate, Maranta oder Maniok. Besonders bevorzugt enthält das erfindungsgemäße Mittel mindestens eine Stärke, die Tapioka Stärke, Kartoffelstärke, Maisstärke oder Reisstärke ist. Gemische der vorgenannten Stärkeverbindungen sind erfindungsgemäß ebenso umfasst. Ganz besonders bevorzugt ist Tapiokastärke.

Die Stärkeverbindung ist in den erfindungsgemäßen Mitteln bevorzugt in einer Menge von 0,05 bis 8,0 Gew.-%, insbesondere von 1,0 bis 7,0 Gew.-%, jeweils bezogen auf das Gewicht des Mittels, enthalten.

Im Rahmen einer weiteren Ausführungsform der Erfindung ist es erfindungsgemäß bevorzugt, wenn der partikelförmig dispergierte Feststoff an der Oberfläche des Feststoffpartikels mit mindestens einem Wasser-in-Öl-Emulgator beschichtet ist.

Besonders geeignete Wasser-in-Öl-Emulgatoren werden bevorzugt ausgewählt aus mindestens einer Verbindung aus der Gruppe, die gebildet wird aus
- Partialester von Polyglycerinen mit n = 2 bis 10 Glycerineinheiten und mit 1 bis 5 gesättigten oder ungesättigten, linearen oder verzweigten, gegebenenfalls hydroxylierten C₈-C₃₀-Fettsäureresten verestert,
- lineare oder verzweigte, gesättigte oder ungesättigte C₁₂-C₃₀-Alkanole, die jeweils mit 1-4 Ethylenoxid-Einheiten pro Molekül verethert sind, welche ausserordentlich bevorzugt sind aus Steareth, Ceteth, Myristeth, Laureth, Trideceth, Arachideth und Beheneth mit jeweils 1-4 Ethylenoxid-Einheiten pro Molekül, insbesondere Steareth-2, Steareth-3, Steareth-4, Ceteth-2, Ceteth-3, Ceteth-4, Myristeth-2, Myristeth-3, Myristeth-4, Laureth-2, Laureth-3, Laureth-4, Trideceth-2, Trideceth-3 und Trideceth-4 sowie Mischungen hiervon;
- lineare gesättigte Alkanole mit 12-30 Kohlenstoffatomen, insbesondere mit 16-22 Kohlenstoffatomen, insbesondere Cetylalkohol, Stearylalkohol, Arachidylalkohol, Behenylalkohol und Lanolinalkohol oder Gemische dieser Alkohole, wie sie bei der technischen Hydrierung von pflanzlichen und tierischen Fettsäuren erhältlich sind;
- Ester und insbesondere Partialester aus einem Polyol mit 2-6 C-Atomen und linearen gesättigten und ungesättigten Fettsäuren mit 12-30, insbesondere 14-22 C-Atomen, die hydroxyliert sein können. Solche Ester oder Partialester sind z. B. die Mono- und Diester von Glycerin oder Ethylenglycol oder die Monoester von Propylenglycol mit linearen gesättigten und ungesättigten C₁₂-C₃₀-Carbonsäuren, die hydroxyliert sein können, insbesondere diejenigen mit Palmitin- und Stearinsäure, die Sorbitanmono-, - di- oder -triester von linearen gesättigten und ungesättigten C₁₂-C₃₀-Carbonsäuren, die hydroxyliert sein können, insbesondere diejenigen von Myristinsäure, Palmitinsäure, Stearinsäure oder von Mischungen dieser Fettsäuren, die Pentaerythritylmono-, -di-, - tri- und -tetraester und die Methylglucosemono- und -diester von linearen, gesättigten und ungesättigten C₁₂-C₃₀-Carbonsäuren, die hydroxyliert sein können, wovon besonders bevorzugt sind die Mono-, Di-, Tri- und Tetraester von Pentaerythrit mit linearen gesättigten Fettsäuren mit 12-30, insbesondere 14-22 Kohlenstoffatomen, die hydroxyliert sein können, sowie Mischungen hiervon, als Konsistenzgeber und/oder Wasserbinder. Erfindungsgemäss besonders bevorzugt sind die Mono- und Diester. Erfindungsgemäss bevorzugte C₁₂-C₃₀-Fettsäurereste sind ausgewählt aus Laurinsäure-, Myristinsäure-, Palmitinsäure-, Stearinsäure-, Arachinsäure- und Behensäure-Resten; besonders bevorzugt ist der Stearinsäurerest;
- Sterine, also Steroide, die am C3-Atom des Steroid-Gerüstes eine Hydroxylgruppe tragen und sowohl aus tierischem Gewebe (Zoosterine, z. B. Cholesterin, Lanosterin) wie auch aus Pflanzen (Phytosterine, z. B. Ergosterin, Stigmasterin, Sitosterin) und aus Pilzen und Hefen (Mykosterine) isoliert werden und die niedrig ethoxyliert (1-5 EO) sein können;
- Alkanole und Carbonsäuren mit jeweils 8-24 C-Atomen, insbesondere mit 16-22 C-Atomen, in der Alkylgruppe und 1-4 Ethylenoxid-Einheiten pro Molekül, die einen HLB-Wert grösser 1,0 und kleiner/gleich 7,0 aufweisen,
- Glycerinmonoether gesättigter und/oder ungesättigter, verzweigter und/oder unverzweigter Alkohole einer Kettenlänge von 8-30, insbesondere 12-18 Kohlenstoffatomen.

Flüssige Partialester von Polyglycerinen mit n = 2 bis 10 Glycerineinheiten und mit 1 bis 5 gesättigten oder ungesättigten, linearen oder verzweigten, gegebenenfalls hydroxylierten C₈-C₃₀-Fettsäureresten verestert, sind erfindungsgemäß besonders bevorzugte Wasser-in-Öl-Emulgatoren zur Auswahl. Ganz besonders bevorzugt wird der Wasser-in-Öl Emulgator ausgewählt aus mindestens einem Emulgator, der 2 bis 6 aneinander kovalent bindende Glyceryleinheiten und mindestens eine (insbesondere verzweigte) Alkylgruppe mit 8 bis 20 Kohlenstoffatomen umfasst.

Die erfindungsgemäßen Mittel weisen zwingend eine Viskosität von 300000 bis 800000 mPas, insbesondere von 400000 bis 600000 mPas (jeweils gemessen bei 20°C, Brookfield, 4 Umdrehungen pro Minute, Spindel TE) auf.

Diese Viskositäten können sich auch ohne Zusatz eines zusätzlichen Verdickungsmittels für die Verdickung von Öl einstellen. Sollte dies nicht der Fall sein, empfiehlt es sich, der Rezeptur ein Verdickungsmittel für die Verdickung von Öl zuzusetzen. Weitere bevorzugte erfindungsgemäße kosmetische Mittel sind daher dadurch gekennzeichnet, dass sie zusätzlich mindestens ein Verdickungsmittel für die Verdickung von Öl enthalten. Diese zusätzlichen Verdickungsmittel für die Verdickung von Öl sind von den vorgenannten zwingend in dem erfindungsgemäßen Mittel enthaltenen Komponenten verschieden.

Es ist erfindungsgemäß zweckmäßig, dass, falls die zusätzlichen Verdickungsmittel für die Verdickung von Öl in dem erfindungsgemäßen kosmetischen Mittel Anwendung finden, besagte Verdickungsmittel nur in einer solchen Menge zugesetzt werden, bis die Viskosität im erfindungsgemäßen Viskositätsbereich liegt.

Erfindungsgemäß bevorzugte Verdickungsmittel für die Verdickung von Öl sind ausgewählt aus hydrophobierten Tonmineralien, Kieselsäuren (insbesondere pyrogenen Kieselsäuren), Ethylene/Propylene/Styrene Copolymeren, Butylene/ Ethylene/Styrene Copolymeren, Dextrinestern und/oder Siliconelastomeren.

Bevorzugte hydrophobierte Tonmineralien sind ausgewählt aus hydrophobierten Montmorilloniten, hydrophobierten Hectoriten und hydrophobierten Bentoniten, besonders bevorzugt aus Disteardimonium Hectorite, Stearalkonium Hectorite, Quaternium-18 Hectorite und Quaternium-18 Bentonite. Die handelsüblichen Verdickungsmittel stellen diese hydrophobierten Tonmineralien in Form eines Gels in einer Ölkomponente, bevorzugt in Cyclomethicone und/oder einer Nichtsilicon-Ölkomponente, wie z. B. Propylencarbonat, bereit. Derartige Gele sind beispielsweise unter der Handelsbezeichnung Bentone^{®} oder Thixogel erhältlich.

Erfindungsgemäß bevorzugte Zusammensetzungen sind dadurch gekennzeichnet, dass sie hydrophobiertes Tonmineral in einer Gesamtmenge von 0,5 - 10 Gew.-%, bevorzugt 1 - 7 Gew.-%, besonders bevorzugt 2 - 6 Gew.-%, außerordentlich bevorzugt 3 - 5 Gew.-%, jeweils bezogen auf das Gesamtgewicht der erfindungsgemäßen Zusammensetzung, enthalten.

Weitere erfindungsgemäß ganz besonders bevorzugte Verdickungsmittel für die Verdickung von Öl sind ausgewählt aus Kieselsäure, insbesondere pyrogene Kieselsäure (z. B. den Handelsprodukten der Aerosil^{®}-Serie von Evonik Degussa). Diese können an deren Oberfläche durch chemische Modifikation hydrophobiert sein (wie z.B. die silylierten Kieselsäuren mit der INCI-Bezeichnung Silica Silylate) was jedoch nicht bevorzugt ist. Erfindungsgemäß besonders bevorzugt geeignete pyrogene Kieselsäure hat die INCI-Bezeichung Silica.

Wird zusätzlich pyrogener Kieselsäure als Verdickungsmittel für die Verdickung von Öl eingesetzt, werden besonders lagerstabile kosmetische Mittel der vorliegenden Erfindung erhalten.

Erfindungsgemäß bevorzugte kosmetische Mittel sind dadurch gekennzeichnet, dass sie Kieselsäure, bevorzugt pyrogene Kieselsäure, in einer Gesamtmenge von 0,1 bis 6 Gew.-%, bevorzugt 0,5 bis 5 Gew.-%, besonders bevorzugt 1,0 bis 4,5 Gew.-%, ganz besonders bevorzugt 1,5 bis 3,0 Gew.-%, außerordentlich bevorzugt 1,9 bis 2,6 Gew.-%, jeweils bezogen auf das Gesamtgewicht der erfindungsgemäßen Zusammensetzung, enthalten.

Weitere erfindungsgemäß einsetzbare lipophile Verdickungsmittel sind ausgewählt aus Ethylene/ Propylene/Styrene Copolymeren und Butylene/Ethylene/Styrene Copolymeren. Besonders bevorugt werden die Copolymere als vorverdicktes öl-basiertes Gel eingesetzt. Derartige Gele sind beispielsweise unter der Handelsbezeichnung Versagel^{®} (ex Penreco) erhältlich. Bevorzugt sind Gele mit Mineralöl, hydriertem Polyisobuten, Isoparaffinen, wie Isohexadecan oder Isododecan, sowie mit Esterölen, insbesondere mit Isopropylpalmitat oder Isopropylmyristat.

Weitere erfindungsgemäß einsetzbare lipophile Verdickungsmittel sind ausgewählt aus Siliconelastomeren. Eine weitere bevorzugte Ausführungsform der Erfindung ist dadurch gekennzeichnet, dass mindestens ein Siliconelastomer, das erhältlich ist durch die Vernetzung eines Organopolysiloxans, das mindestens 2 C₂ - C₁₀-Alkenylgruppen mit terminaler Doppelbindung in jedem Molekül enthält, mit einem Organopolysiloxan, das mindestens 2 Silicon-gebundene Wasserstoffatome in jedem Molekül aufweist, enthalten ist.

Erfindungsgemäß besonders bevorzugte Organopolysiloxane mit mindestens 2 C₂ - C₁₀ - AlkenylGruppen mit terminaler Doppelbindung im Molekül sind ausgewählt aus Methylvinylsiloxanen, Methylvinylsiloxan-Dimethylsiloxan-Copolymeren, Dimethylpolysiloxanen mit Dimethylvinylsiloxy-Endgruppen, Dimethylsiloxan-Methylphenylsiloxan-Copolymeren mit Dimethylvinylsiloxy-Endgruppen, Dimethylsiloxan-Diphenylsiloxan-Methylvinylsiloxan-Copolymeren mit Dimethylvinylsiloxy-Endgruppen, Dimethylsiloxan-Methylvinylsiloxan-Copolymeren mit Trimethylsiloxy-Endgruppen, Dimethylsiloxan-Methylphenylsiloxan-Methylvinylsiloxan-Copolymeren mit Trimethylsiloxy-Endgruppen, Methyl-(3,3,3-trifluoropropyl)-polysiloxanen mit Dimethylvinylsiloxy-Endgruppen und Dimethylsiloxan-Methyl-(3,3,3-trifluoropropyl)-siloxan-Copolymeren mit Dimethylvinylsiloxy-Endgruppen.

Erfindungsgemäß besonders bevorzugte vernetzende Organopolysiloxane mit mindestens zwei Silicon-gebundenen Wasserstoffatomen sind ausgewählt aus Methylhydrogenpolysiloxanen mit Trimethylsiloxy-Endgruppen, Dimethylsiloxan-Methylhydrogensiloxan-Copolymeren mit Trimethylsiloxy-Endgruppen und cyclischen Dimethylsiloxan-Methylhydrogen-siloxan-Copolymeren.

Erfindungsgemäß besonders bevorzugte Siliconelastomere, die als Rohstoff bereits in einem bei Raumtemperatur unter Normalbedingungen flüssigen Silicon vorgequollen vorliegen und ein Silicon-basiertes Gel darstellen, sind kommerziell erhältlich, beispielsweise unter den Handelsnamen Dow Corning 9040 Silicone Elastomer Blend (ein Cyclomethicone (and) Dimethicone Crosspolymer von Dow Corning; Silikonelastomergehalt 12 - 13 Gew.-%), SFE 168, ein Cyclomethicone (and) Dimethicone/Vinyl Dimethicone Crosspolymer von GE Silicones, Vinyl Dimethicone Crosspolymere, enthalten in KSG-15 (Cyclomethicone (and) Dimethicone/Vinyl Dimethicone Crosspolymer, Siliconelastomergehalt 4 - 10 Gew.-%), KSG-16 (Dimethicone (and) Dimethicone/Vinyl Dimethicone Crosspolymer, Siliconelastomergehalt 20 - 30 Gew.-%), KSG-17 (Cyclomethicone (and) Dimethicone/Vinyl Dimethicone Crosspolymer), KSG-18 (Phenyl Trimethicone (and) Dimethicone/Phenyl Vinyl Dimethicone Crosspolymer, Siliconelastomergehalt 10 - 20 Gew.-%); and KSG-20, erhältlich von Shin Etsu Silicones of America (Akron, Ohio), und von Grant Industries Inc. (Elmwood Park, NJ) die Produkte aus der Gransil^{®}-Serie, insbesondere Gransil SR-CYC (Cyclomethicone and Stearyl-Vinyl/hydromethylsiloxane Copolymer), Gransil^{®} RPS Gel (INCI-Bezeichnung: Cyclopentasiloxane and Polysilicone-11), Gransil^{®} GCM-4 (INCI-Bezeichnung: Cyclotetrasiloxane and Polysilicone-11), Gransil^{®}GCM-5 (INCI-Bezeichnung: Cyclopentasiloxane and Polysilicone-11), Gransil^{®} RPS (INCI-Bezeichnung: Cyclopentasiloxane and Polysilicone-11), GI-CD 10 (INCI-Bezeichnung: Cyclopentasiloxane (and) Stearoxymethicone/Dimethicone Copolymer (and) Dimethicone), Gransil^{®} IDS (INCI-Bezeichnung: Isododecane (and) Cyclotetrasiloxane (and) Polysilicone-11), Gransi^{®}PC-12 (INCI-Bezeichnung: Isododecane (and) Polysilicone-11), Gransil^{®}IDS-5 (INCI-Bezeichnung: Isododecane (and) Cyclopentasiloxane (and) Polysilicone-11), Gransil^{®}APK-1 (INCI-Bezeichnung: Dimethicone and Cyclopentasiloxane and Polysilicone-11 and Nylon-12 and Methyl Methacrylate/Acrylonitrile Copolymer and PEG-10 Dimethicone and Polysorbate-40 and Isohexadecane and Ammonium Polyacryloyldimethyl Taurate), Gransil^{®}DMCM-5 (INCI-Bezeichnung: Dimethicone and Cyclopentasiloxane and Polysilicone-11), Gransil^{®}DMG-6 mit Dimethicone (6 cSt) (INCI-Bezeichnung: Dimethicone and Polysilicone-11), Gransil^{®}DMG-20 mit Dimethicone (20 cSt) (INCI-Bezeichnung: Dimethicone and Polysilicone-11), Gransil^{®} AM-8 Gel (INCI-Bezeichnung: Caprylyl Methicone and Cyclopentasiloxane and Polysilicone-11), Gransil^{®} DM 5 mit Dimethicone (5 cSt) (INCI-Bezeichnung: Dimethicone and Polysilicone-11), Gransil^{®} DMID (INCI-Bezeichnung: Dimethicone and Isododecane and Polysilicone-11), Gransil^{®} PM (INCI-Bezeichnung: Phenyl Trimethicone and Polysilicone-11), Gransil^{®} ININ (INCI-Bezeichnung: Isononyl Isononanoate (and) Polysilicone-11).

Ebenfalls mit Vorzug in den erfindungsgemäßen Zusammensetzungen einsetzbar sind Siliconelastomere, die als Rohstoff bereits in einem bei Raumtemperatur unter Normalbedingungen flüssigen Siliconöl, Fett oder Wachs, vorgequollen vorliegen und ein Silicon-/Nicht-Silicon-basiertes Gel darstellen. Derartige Siliconelastomer-Zusammensetzungen sind ebenfalls kommerziell erhältlich, beispielsweise unter den Handelsnamen Gransil^{®} MLB (INCI-Bezeichnung: Cyclopentasiloxane and Polysilicone-11 and Beeswax), Gransil^{®} PS (INCI-Bezeichnung: Cyclotetrasiloxane and Polysilicone-11 and Petrolatum), Gransil^{®} PS-5 (INCI-Bezeichnung: Cyclopentasiloxane and Polysilicone-11 and Petrolatum), Gransil^{®} DMG-20 P mit Dimethicone (20 cSt) und Petrolatum (INCI-Bezeichnung: Dimethicone and Polysilicone-11 and Petrolatum), Gransil^{®} RJO (INCI-Bezeichnung: Cyclopentasiloxane and Polysilicone-11 and Jojoba Oil), Gransil^{®} LANO (INCI-Bezeichnung: Cyclopentasiloxane and Polysilicone-11 and Lanolin), Gransil^{®} OHS-5 (INCI-Bezeichnung: Cyclopentasiloxane and Polysilicone-11 and Octyl Hydroxystearate) und Gransil^{®} DML (INCI-Bezeichnung: Dimethicone (and) Neopentyl Glycol Diheptanoate (and) Polysilicone-11).

Eine weitere bevorzugte Ausführungsform der Erfindung ist dadurch gekennzeichnet, dass das Siliconelastomer durch die Vernetzung eines Organopolysiloxans, das mindestens 2 C₂ - C₁₀-Alkenylgruppen mit terminaler Doppelbindung in jedem Molekül enthält, mit mindestens einem alpha, omega-Dien erhältlich ist. Erfindungsgemäß besonders bevorzugte vernetzende alpha, omega-Diene weisen die Formel CH₂=CH(CH₂)ₓCH=CH₂ mit x = 1 - 20 auf. Besonders bevorzugte alpha, omega-Diene sind ausgewählt aus 1,4-Pentadien, 1,5-Hexadien, 1,6-Heptadien, 1,7-Octadien, 1,8-Nonadien, 1,11-Dodecadien, 1,13-Tetradecadien und 1,19- Eicosadien.

Erfindungsgemäß bevorzugte Zusammensetzungen sind dadurch gekennzeichnet, dass sie mindestens ein Siliconelastomer in einer Gesamtmenge von 0,05 - 3 Gew.-%, bevorzugt 0,1 - 2 Gew.-%, besonders bevorzugt 0,2 - 1,0 Gew.-%, außerordentlich bevorzugt 0,3 - 0,5 Gew.-%, jeweils bezogen auf das Gesamtgewicht der erfindungsgemäßen Zusammensetzung, enthalten.

Üblicherweise weisen ölbasierte Zusammensetzungen, die partikuläre Feststoffe enthalten bei 20°C eine Dichte von größer 1,2 g/cm³ auf. Die erfindungsgemäßen Mittel besitzen bei 20°C eine Dichte von 0,95 bis 1,10 g/cm³. Die Dichte der erfindungsgemäßen Mittel ist folglich signifikant niedriger, als bei üblichen vergleichbaren Zusammensetzungen des Standes der Technik.

Der Fachmann kennt zuverlässige Messmethoden zur reproduzierbaren und eindeutigen Bestimmung der Dichte. Im Rahmen der vorliegenden Anmeldung wurde die Dichte mittels digitalem Dichtemesser (z.B. Chempro DMA 4100M oder Mettler-Toledo Density Meter DM40) bestimmt. Das Messprinzip beruht auf der sogenannten Biegeschwingungs-Methode, bei der die Substanz in ein an den Enden offenes U-förmiges Glasrohr des Messgerätes eingefüllt wird. Das besagte Glasrohr wird bei konstanter Temperatur gehalten (hier 20°C), elektronisch in Schwingung versetzt und die Eigenfrequenz der Schwingung ermittelt. Diese Eigenfrequenz ist charakteristisch für die Dichte der Probe.

Die Senkung der Dichte wird besonders bevorzugt durch Dispersion von Gas erreicht. Erfindungsgemäß besonders bevorzugte kosmetische Mittel enthalten daher dispergiertes Gas.

Als erfindungsgemäß bevorzugt geeignete Gase gelten Luft, Stickstoff, Sauerstoff, Kohlendioxid, Argon, Distickstoffoxid (besonders bevorzugt Luft).

Gas kann beispielsweise durch Einblasen, Umpumpen, Extrudieren oder Rühren in das erfindungsgemäße kosmetische Mittel eingearbeitet werden. Besonders bevorzugt wird die Gaskomponente durch einen Homogenisator in das erfindungsgemäße kosmetische Mittel dispergiert.

Eine besonders bevorzugte Ausführungsform der Erfindung wird durch folgende Punkte charakterisiert:
(A): Kosmetisches Mittel, enthaltend jeweils bezogen auf das Gesamtgewicht des Mittels
   - 0 bis 3 Gew.-%, insbesondere 0 bis 2 Gew.-%, freies Wasser,
   - ein oder mehrere flüssige Öle in einer Gesamtmenge von 40 bis 80 Gew.-%, insbesondere von 50 bis 75 Gew.-%, insbesondere von 60 bis 70 Gew.-%,
   - mindestens ein Wachs mit einem Schmelzpunkt in einem Bereich von 30°C bis 60°C, besonders bevorzugt in einem Bereich von 30°C bis 50°C, ganz besonders bevorzugt in einem Bereich von 30°C bis 40°C,
   - partikelförmig dispergierten Feststoff,
   - gegebenenfalls weitere Inhaltsstoffe,
   wobei
   - das kosmetische Mittel eine Viskosität von 300000 bis 800000 mPas, insbesondere von 400000 bis 600000 mPas (jeweils gemessen bei 20°C, Brookfield, 4 Umdrehungen pro Minute, Spindel TE) besitzt,
   - das kosmetische Mittel bei 20°C eine Dichte von 0,95 bis 1,10 g/cm³ hat.
(B): Kosmetisches Mittel, enthaltend jeweils bezogen auf das Gesamtgewicht des Mittels
   - 0 bis 3 Gew.-%, insbesondere 0 bis 2 Gew.-%, freies Wasser,
   - ein oder mehrere flüssige Öle in einer Gesamtmenge von 40 bis 80 Gew.-%, insbesondere von 50 bis 75 Gew.-%, insbesondere von 60 bis 70 Gew.-%,
   - mindestens ein Wachs mit einem Schmelzpunkt in einem Bereich von 30°C bis 60°C, besonders bevorzugt in einem Bereich von 30°C bis 50°C, ganz besonders bevorzugt in einem Bereich von 30°C bis 40°C,
   - partikelförmig dispergierten Feststoff,
   - gegebenenfalls weitere Inhaltsstoffe,
   wobei
   - das kosmetische Mittel eine Viskosität von 300000 bis 800000 mPas, insbesondere von 400000 bis 600000 mPas (jeweils gemessen bei 20°C, Brookfield, 4 Umdrehungen pro Minute, Spindel TE) besitzt,
   - das kosmetische Mittel bei 20°C eine Dichte von 0,95 bis 1,10 g/cm³ hat.
(C): Kosmetisches Mittel, enthaltend jeweils bezogen auf das Gesamtgewicht des Mittels
   - 0 bis 3 Gew.-%, insbesondere 0 bis 2 Gew.-%, freies Wasser,
   - ein oder mehrere flüssige Öle in einer Gesamtmenge von 40 bis 80 Gew.-%, insbesondere von 50 bis 75 Gew.-%, insbesondere von 60 bis 70 Gew.-%,
   - mindestens ein Wachs mit einem Schmelzpunkt in einem Bereich von 30°C bis 60°C, besonders bevorzugt in einem Bereich von 30°C bis 50°C, ganz besonders bevorzugt in einem Bereich von 30°C bis 40°C,
   - partikelförmig dispergierten Feststoff in einer Gesamtmenge von 10 bis 30 Gew.-%,
   - gegebenenfalls weitere Inhaltsstoffe,
   wobei
   - das kosmetische Mittel eine Viskosität von 300000 bis 800000 mPas, insbesondere von 400000 bis 600000 mPas (jeweils gemessen bei 20°C, Brookfield, 4 Umdrehungen pro Minute, Spindel TE) besitzt,
   - das kosmetische Mittel bei 20°C eine Dichte von 0,95 bis 1,10 g/cm³ hat.
(D): Kosmetisches Mittel, enthaltend jeweils bezogen auf das Gesamtgewicht des Mittels
   - 0 bis 3 Gew.-%, insbesondere 0 bis 2 Gew.-%, freies Wasser,
   - ein oder mehrere flüssige Öle in einer Gesamtmenge von 40 bis 80 Gew.-%, insbesondere von 50 bis 75 Gew.-%, insbesondere von 60 bis 70 Gew.-%,
   - mindestens ein Wachs mit einem Schmelzpunkt in einem Bereich von 30°C bis 60°C, besonders bevorzugt in einem Bereich von 30°C bis 50°C, ganz besonders bevorzugt in einem Bereich von 30°C bis 40°C, in einer Gesamtmenge von 0,05 bis 8,0 Gew.-%, besonders bevorzugt von 0,1 bis 5,0 Gew.-%, ganz besonders bevorzugt von 0,5 bis 3,0 Gew.-%,
   - partikelförmig dispergierten Feststoff,
   - gegebenenfalls weitere Inhaltsstoffe,
   wobei
   - das kosmetische Mittel eine Viskosität von 300000 bis 800000 mPas, insbesondere von 400000 bis 600000 mPas (jeweils gemessen bei 20°C, Brookfield, 4 Umdrehungen pro Minute, Spindel TE) besitzt,
   - das kosmetische Mittel bei 20°C eine Dichte von 0,95 bis 1,10 g/cm³ hat.
(E): Kosmetisches Mittel, enthaltend jeweils bezogen auf das Gesamtgewicht des Mittels
   - 0 bis 3 Gew.-%, insbesondere 0 bis 2 Gew.-%, freies Wasser,
   - ein oder mehrere flüssige Öle in einer Gesamtmenge von 40 bis 80 Gew.-%, insbesondere von 50 bis 75 Gew.-%, insbesondere von 60 bis 70 Gew.-%, wobei als flüssiges Öl zumindest enthalten ist:
      - mindestens ein Ester der Formel (I) 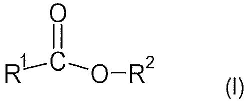 worin R¹ und R² unabhängig voneinander für eine lineare oder verzweigte Kohlenwasserstoffgruppe mit 3 bis 20 Kohlenstoffatomen stehen, und
      - mindestens ein Silikonöl,
   - mindestens ein Wachs mit einem Schmelzpunkt in einem Bereich von 30°C bis 60°C, besonders bevorzugt in einem Bereich von 30°C bis 50°C, ganz besonders bevorzugt in einem Bereich von 30°C bis 40°C,
   - partikelförmig dispergierten Feststoff,
   - gegebenenfalls weitere Inhaltsstoffe,
   wobei
   - das kosmetische Mittel eine Viskosität von 300000 bis 800000 mPas, insbesondere von 400000 bis 600000 mPas (jeweils gemessen bei 20°C, Brookfield, 4 Umdrehungen pro Minute, Spindel TE) besitzt,
   - das kosmetische Mittel bei 20°C eine Dichte von 0,95 bis 1,10 g/cm³ hat.
(F): Kosmetisches Mittel, enthaltend jeweils bezogen auf das Gesamtgewicht des Mittels
   - 0 bis 3 Gew.-%, insbesondere 0 bis 2 Gew.-%, freies Wasser,
   - ein oder mehrere flüssige Öle in einer Gesamtmenge von 40 bis 80 Gew.-%, insbesondere von 50 bis 75 Gew.-%, insbesondere von 60 bis 70 Gew.-%, wobei als flüssiges Öl zumindest enthalten ist:
      - mindestens ein Ester der Formel (I) 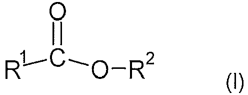 worin R¹ und R² unabhängig voneinander für eine lineare oder verzweigte Kohlenwasserstoffgruppe mit 3 bis 20 Kohlenstoffatomen stehen, und
      - mindestens ein Silikonöl,
   - mindestens ein Wachs mit einem Schmelzpunkt in einem Bereich von 30°C bis 60°C, besonders bevorzugt in einem Bereich von 30°C bis 50°C, ganz besonders bevorzugt in einem Bereich von 30°C bis 40°C,
   - partikelförmig dispergierten Feststoff,
   - gegebenenfalls weitere Inhaltsstoffe,
   wobei
   - das kosmetische Mittel eine Viskosität von 300000 bis 800000 mPas, insbesondere von 400000 bis 600000 mPas (jeweils gemessen bei 20°C, Brookfield, 4 Umdrehungen pro Minute, Spindel TE) besitzt,
   - das kosmetische Mittel bei 20°C eine Dichte von 0,95 bis 1,10 g/cm³ hat.
(G): Kosmetisches Mittel, enthaltend jeweils bezogen auf das Gesamtgewicht des Mittels
   - 0 bis 3 Gew.-%, insbesondere 0 bis 2 Gew.-%, freies Wasser,
   - ein oder mehrere flüssige Öle in einer Gesamtmenge von 40 bis 80 Gew.-%, insbesondere von 50 bis 75 Gew.-%, insbesondere von 60 bis 70 Gew.-%, wobei als flüssiges Öl zumindest enthalten ist:
      - mindestens ein Ester der Formel (I) 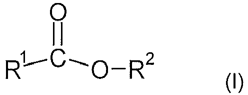 worin R¹ und R² unabhängig voneinander für eine lineare oder verzweigte Kohlenwasserstoffgruppe mit 3 bis 20 Kohlenstoffatomen stehen, und
      - mindestens ein Silikonöl,
   - mindestens ein Wachs mit einem Schmelzpunkt in einem Bereich von 30°C bis 60°C, besonders bevorzugt in einem Bereich von 30°C bis 50°C, ganz besonders bevorzugt in einem Bereich von 30°C bis 40°C,
   - partikelförmig dispergierten Feststoff in einer Gesamtmenge von 10 bis 30 Gew.-%,
   - gegebenenfalls weitere Inhaltsstoffe,
   wobei
   - das kosmetische Mittel eine Viskosität von 300000 bis 800000 mPas, insbesondere von 400000 bis 600000 mPas (jeweils gemessen bei 20°C, Brookfield, 4 Umdrehungen pro Minute, Spindel TE) besitzt,
   - das kosmetische Mittel bei 20°C eine Dichte von 0,95 bis 1,10 g/cm³ hat.
(H): Kosmetisches Mittel, enthaltend jeweils bezogen auf das Gesamtgewicht des Mittels
   - 0 bis 3 Gew.-%, insbesondere 0 bis 2 Gew.-%, freies Wasser,
   - ein oder mehrere flüssige Öle in einer Gesamtmenge von 40 bis 80 Gew.-%, insbesondere von 50 bis 75 Gew.-%, insbesondere von 60 bis 70 Gew.-%, wobei als flüssiges Öl zumindest enthalten ist:
      - mindestens ein Ester der Formel (I) 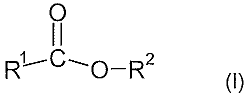 worin R¹ und R² unabhängig voneinander für eine lineare oder verzweigte Kohlenwasserstoffgruppe mit 3 bis 20 Kohlenstoffatomen stehen, und
      - mindestens ein Silikonöl,
   - mindestens ein Wachs mit einem Schmelzpunkt in einem Bereich von 30°C bis 60°C, besonders bevorzugt in einem Bereich von 30°C bis 50°C, ganz besonders bevorzugt in einem Bereich von 30°C bis 40°C, in einer Gesamtmenge von 0,05 bis 8,0 Gew.-%, besonders bevorzugt von 0,1 bis 5,0 Gew.-%, ganz besonders bevorzugt von 0,5 bis 3,0 Gew.-%,
   - partikelförmig dispergierten Feststoff,
   - gegebenenfalls weitere Inhaltsstoffe,
   wobei
   - das kosmetische Mittel eine Viskosität von 300000 bis 800000 mPas, insbesondere von 400000 bis 600000 mPas (jeweils gemessen bei 20°C, Brookfield, 4 Umdrehungen pro Minute, Spindel TE) besitzt,
   - das kosmetische Mittel bei 20°C eine Dichte von 0,95 bis 1,10 g/cm³ hat.
(I): Kosmetisches Mittel, enthaltend jeweils bezogen auf das Gesamtgewicht des Mittels
   - 0 bis 3 Gew.-%, insbesondere 0 bis 2 Gew.-%, freies Wasser,
   - ein oder mehrere flüssige Öle in einer Gesamtmenge von 40 bis 80 Gew.-%, insbesondere von 50 bis 75 Gew.-%, insbesondere von 60 bis 70 Gew.-%, wobei als flüssiges Öl zumindest enthalten ist:
      - ein oder mehrere Ester der Formel (I) 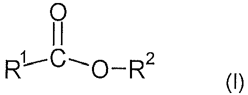 worin R¹ und R² unabhängig voneinander für eine lineare oder verzweigte Kohlenwasserstoffgruppe mit 3 bis 20 Kohlenstoffatomen stehen, in einer Gesamtmenge von 1 bis 20, insbesondere von 5 bis 15 Gew.-%,
      - Silikonöl in einer Gesamtmenge von 30 bis 60 Gew.-%, insbesondere von 40 bis 60 Gew.-%,
   - mindestens ein Wachs mit einem Schmelzpunkt in einem Bereich von 30°C bis 60°C, besonders bevorzugt in einem Bereich von 30°C bis 50°C, ganz besonders bevorzugt in einem Bereich von 30°C bis 40°C,
   - partikelförmig dispergierten Feststoff,
   - gegebenenfalls weitere Inhaltsstoffe,
   wobei
   - das kosmetische Mittel eine Viskosität von 300000 bis 800000 mPas, insbesondere von 400000 bis 600000 mPas (jeweils gemessen bei 20°C, Brookfield, 4 Umdrehungen pro Minute, Spindel TE) besitzt,
   - das kosmetische Mittel bei 20°C eine Dichte von 0,95 bis 1,10 g/cm³ hat.
(J): Kosmetisches Mittel, enthaltend jeweils bezogen auf das Gesamtgewicht des Mittels
   - 0 bis 3 Gew.-%, insbesondere 0 bis 2 Gew.-%, freies Wasser,
   - ein oder mehrere flüssige Öle in einer Gesamtmenge von 40 bis 80 Gew.-%, insbesondere von 50 bis 75 Gew.-%, insbesondere von 60 bis 70 Gew.-%, wobei als flüssiges Öl zumindest enthalten ist:
      - ein oder mehrere Ester der Formel (I) 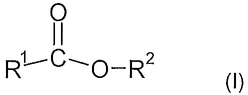 worin R¹ und R² unabhängig voneinander für eine lineare oder verzweigte Kohlenwasserstoffgruppe mit 3 bis 20 Kohlenstoffatomen stehen, in einer Gesamtmenge von 1 bis 20, insbesondere von 5 bis 15 Gew.-%,
      - Silikonöl in einer Gesamtmenge von 30 bis 60 Gew.-%, insbesondere von 40 bis 60 Gew.-%,
   - mindestens ein Wachs mit einem Schmelzpunkt in einem Bereich von 30°C bis 60°C, besonders bevorzugt in einem Bereich von 30°C bis 50°C, ganz besonders bevorzugt in einem Bereich von 30°C bis 40°C,
   - partikelförmig dispergierten Feststoff,
   - gegebenenfalls weitere Inhaltsstoffe,
   wobei
   - das kosmetische Mittel eine Viskosität von 300000 bis 800000 mPas, insbesondere von 400000 bis 600000 mPas (jeweils gemessen bei 20°C, Brookfield, 4 Umdrehungen pro Minute, Spindel TE) besitzt,
   - das kosmetische Mittel bei 20°C eine Dichte von 0,95 bis 1,10 g/cm³ hat.
(K): Kosmetisches Mittel, enthaltend jeweils bezogen auf das Gesamtgewicht des Mittels
   - 0 bis 3 Gew.-%, insbesondere 0 bis 2 Gew.-%, freies Wasser,
   - ein oder mehrere flüssige Öle in einer Gesamtmenge von 40 bis 80 Gew.-%, insbesondere von 50 bis 75 Gew.-%, insbesondere von 60 bis 70 Gew.-%, wobei als flüssiges Öl zumindest enthalten ist:
      - ein oder mehrere Ester der Formel (I) 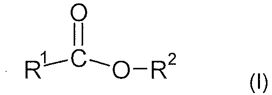 worin R¹ und R² unabhängig voneinander für eine lineare oder verzweigte Kohlenwasserstoffgruppe mit 3 bis 20 Kohlenstoffatomen stehen, in einer Gesamtmenge von 1 bis 20, insbesondere von 5 bis 15 Gew.-%,
      - Silikonöl in einer Gesamtmenge von 30 bis 60 Gew.-%, insbesondere von 40 bis 60 Gew.-%,
   - mindestens ein Wachs mit einem Schmelzpunkt in einem Bereich von 30°C bis 60°C, besonders bevorzugt in einem Bereich von 30°C bis 50°C, ganz besonders bevorzugt in einem Bereich von 30°C bis 40°C,
   - partikelförmig dispergierten Feststoff in einer Gesamtmenge von 10 bis 30 Gew.-%,
   - gegebenenfalls weitere Inhaltsstoffe,
   wobei
   - das kosmetische Mittel eine Viskosität von 300000 bis 800000 mPas, insbesondere von 400000 bis 600000 mPas (jeweils gemessen bei 20°C, Brookfield, 4 Umdrehungen pro Minute, Spindel TE) besitzt,
   - das kosmetische Mittel bei 20°C eine Dichte von 0,95 bis 1,10 g/cm³ hat.
(L): Kosmetisches Mittel, enthaltend jeweils bezogen auf das Gesamtgewicht des Mittels
   - 0 bis 3 Gew.-%, insbesondere 0 bis 2 Gew.-%, freies Wasser,
   - ein oder mehrere flüssige Öle in einer Gesamtmenge von 40 bis 80 Gew.-%, insbesondere von 50 bis 75 Gew.-%, insbesondere von 60 bis 70 Gew.-%, wobei als flüssiges Öl zumindest enthalten ist:
      - ein oder mehrere Ester der Formel (I) 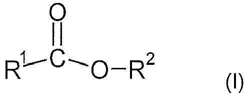 worin R¹ und R² unabhängig voneinander für eine lineare oder verzweigte Kohlenwasserstoffgruppe mit 3 bis 20 Kohlenstoffatomen stehen, in einer Gesamtmenge von 1 bis 20, insbesondere von 5 bis 15 Gew.-%,
      - Silikonöl in einer Gesamtmenge von 30 bis 60 Gew.-%, insbesondere von 40 bis 60 Gew.-%,
   - mindestens ein Wachs mit einem Schmelzpunkt in einem Bereich von 30°C bis 60°C, besonders bevorzugt in einem Bereich von 30°C bis 50°C, ganz besonders bevorzugt in einem Bereich von 30°C bis 40°C, in einer Gesamtmenge von 0,05 bis 8,0 Gew.-%, besonders bevorzugt von 0,1 bis 5,0 Gew.-%, ganz besonders bevorzugt von 0,5 bis 3,0 Gew.-%,
   - partikelförmig dispergierten Feststoff,
   - gegebenenfalls weitere Inhaltsstoffe,
   wobei
   - das kosmetische Mittel eine Viskosität von 300000 bis 800000 mPas, insbesondere von 400000 bis 600000 mPas (jeweils gemessen bei 20°C, Brookfield, 4 Umdrehungen pro Minute, Spindel TE) besitzt,
   - das kosmetische Mittel bei 20°C eine Dichte von 0,95 bis 1,10 g/cm³ hat.
(N): Kosmetisches Mittel, enthaltend jeweils bezogen auf das Gesamtgewicht des Mittels
   - 0 bis 3 Gew.-%, insbesondere 0 bis 2 Gew.-%, freies Wasser,
   - ein oder mehrere flüssige Öle in einer Gesamtmenge von 50 bis 75 Gew.-%, insbesondere von 60 bis 70 Gew.-%, wobei als flüssiges Öl zumindest enthalten ist:
      - ein oder mehrere Ester der Formel (I) 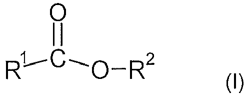 worin R¹ und R² unabhängig voneinander für eine lineare oder verzweigte Kohlenwasserstoffgruppe mit 3 bis 20 Kohlenstoffatomen stehen, in einer Gesamtmenge von 1 bis 15, insbesondere von 5 bis 15 Gew.-%,
      - Silikonöl in einer Gesamtmenge von 30 bis 60 Gew.-%, insbesondere von 40 bis 60 Gew.-%,
   - mindestens ein Wachs mit einem Schmelzpunkt in einem Bereich von 30°C bis 60°C, besonders bevorzugt in einem Bereich von 30°C bis 50°C, ganz besonders bevorzugt in einem Bereich von 30°C bis 40°C, in einer Gesamtmenge von 0,05 bis 8,0 Gew.-%, besonders bevorzugt von 0,1 bis 5,0 Gew.-%, ganz besonders bevorzugt von 0,5 bis 3,0 Gew.-%,
   - partikelförmig dispergierten Feststoff in einer Gesamtmenge von 10 bis 30 Gew.-%,
   - gegebenenfalls weitere Inhaltsstoffe,
   wobei
   - das kosmetische Mittel eine Viskosität von 300000 bis 800000 mPas, insbesondere von 400000 bis 600000 mPas (jeweils gemessen bei 20°C, Brookfield, 4 Umdrehungen pro Minute, Spindel TE) besitzt,
   - das kosmetische Mittel bei 20°C eine Dichte von 0,95 bis 1,10 g/cm³ hat.

Es ist erfindungsgemäß bevorzugt, wenn die Ausführungsformen (A) bis (N) jeweils zusätzlich mindestens ein Verdickungsmittel für die Verdickung von Öl (insbesondere Kieselsäure, besonders bevorzugt pyrogene Kieselsäure) enthält.

Ein zweiter Erfindungsgegenstand ist ein Herstellungsverfahren für kosmetische Mittel, worin
- Wachs in einer flüssigen Ölphase unter Bildung einer Wachslösung gelöst wird,
- in dieser Wachslösung Feststoff in Partikelform dispergiert wird
- gegebenenfalls weitere Inhaltsstoffe zur Wachslösung gegeben werden,
- gegebenenfalls a) gemeinsam mit dem Feststoff in Partikelform oder
   b) gemeinsam mit den weiteren Inhaltsstoffen oder
   c) abschließend gesondert
   mindestens ein Verdickungsmittel für die Verdickung von Öl zugefügt wird,
und in die resultierende Mischung oder während des Mischvorganges gasförmiger Stoff dispergiert wird, bis das kosmetische Mittel eine Dichte (gemessen bei 20°C) von 0.95 bis 1.10 g/cm³ besitzt.

Der gasförmige Stoff kann beispielsweise durch Einblasen, Umpumpen, Extrudieren oder Rühren in das erfindungsgemäße kosmetische Mittel eingearbeitet werden. Besonders bevorzugt wird der gasförmige Stoff durch einen Homogenisator in das erfindungsgemäße kosmetische Mittel dispergiert.

Als erfindungsgemäß bevorzugt geeigneter gasförmiger Stoff gilt Luft, Stickstoff, Sauerstoff, Kohlendioxid, Argon, Distickstoffoxid, Mischungen zweier oder mehrerer der vorgenannten Gase (besonders bevorzugt Luft).

Das erfindungsgemäße kosmetische Mittel wird besonders bevorzugt nach folgendem Verfahren, umfassend folgende Schritte, hergestellt:
i) in einem Rührgefäß mit Rührer (und bevorzugt mit Homogenisator) wird bei einer Temperatur von mindestens 40°C, insbesondere von 40°C bis 45°C, Wachs in einer flüssigen Ölkomponente unter Rühren gelöst,
ii) die resultierende Wachslösung wird auf eine Temperatur von höchstens 25°C heruntergekühlt,
iii) bei einer Temperatur von höchstens 25°C wird in die Wachslösung Feststoff in Partikelform unter Rühren (bevorzugt unter Verwendung eines Homogenisators) dispergiert,
iv) bei einer Temperatur von höchstens 25°C werden weitere Inhaltsstoffe unter Rühren zugegeben,
v) bei einer Temperatur von 20°-25°C wird unter Rühren mindestens ein Verdickungsmittel für die Verdickung von Öl zugegeben, insbesondere Silika,
vi) im Rührgefäß wird ein Gasdruck auf einen Überdruck im Bereich von 10 bis 250 mbar angelegt und die Mischung für einen Zeitraum von 10 bis 60 Minuten, insbesondere von 20 bis 40 Minuten, gerührt.

Zur Herstellung eignet sich beispielsweise ein Mischer Symex 1000 von der Firma Schröder & Boos GmbH & Co. KG, Deutschland.

Es ist erfindungsgemäß bevorzugt als flüssige Ölkomponente gemäß Schritt i) einen oder mehrere Ester der Formel (I) 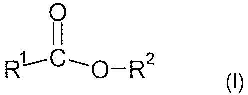 worin R¹ und R² unabhängig voneinander für eine lineare oder verzweigte Kohlenwasserstoffgruppe mit 3 bis 20 Kohlenstoffatomen stehen, bevorzugt in einer Gesamtmenge von 1 bis 20, insbesondere von 5 bis 15 Gew.-%, einzusetzen.

Es ist erfindungsgemäß bevorzugt nach oder während Schritt ii) (bevorzugt zwischen Schritt ii) und vor Schritt iii) ) mindestens ein Silikonöl (bevorzugt in einer Menge von 30 bis 60 Gew.-%, insbesondere von 40 bis 60 Gew.-%) zuzugeben.

Ferner sind alle bevorzugten Ausführungsformen der Parameter der erfindungsgemäßen Mittel des ersten Erfindungsgegenstandes auch für den zweiten Erfindungsgegenstand *mutatis mutandis* bevorzugt.

Ein dritter Erfindungsgegenstand ist die Verwendung eines kosmetischen Mittels des ersten Erfindungsgegenstandes als Hautkosmetikum.

### Beispiele

### 1.0 Herstellung Mousse

Es wurde folgendes kosmetisches Mittel gemäß Erfindung hergestellt:

**Tabelle 1: Mousse**

| **Nr.** | **Inhaltsstoff** | **Menge in Gew.-%** |
|---|---|---|
| **1** | 2-Ethylhexylpalmitat | 10,00 |
| **1** | Cocoglycerides (Wachs, Schmelzpunkt 30-33°C) | 2,00 |
| **2** | Dimethicone | ad 100 |
| **2** | Dimethicone Crosspolymer | 5,67 |
| **2** | Cyclomethicone | 0,32 |
| **2** | Caprylic/Capric Triglyceride | 1,90 |
| **3** | Polyglyceryl-3 Diisostearate | 1,00 |
| **3** | Titanium Dioxide (Cl 77891) | 4,00 |
| **3** | Iron Oxides (Cl 77492) | 0,45 |
| **3** | Iron Oxides (Cl 77491) | 0,40 |
| **3** | Iron Oxides (Cl 77499) | 0,12 |
| **3** | Talc | 10,00 |
| **3** | Tapioca Starch | 4,70 |
| **4** | Pentylene Glycol | 0,04 |
| **4** | Wasser | 0,60 |
| **4** | Citric Acid | 0,05 |
| **4** | Sodium Carbonate | 0,05 |
| **4** | Parfum | 0,30 |
| **4** | Hydrolyzed Soy Protein | 0,10 |
| **4** | Spilanthes Acmella Flower Extract | 0,05 |
| **5** | Silica (pyrogen) | 3,00 |

Dichte (20°C): 1,02 g/cm³ Viskosität (Brookfield, 20°C, 4 rpm, Spindel TE): 400000 - 600000 mPas, In einem Rührgefäß (Symex 1000 der Firma Schröder & Boos GmbH & Co. KG, Deutschland) mit Rührer und Homogenisator wurde bei einer Temperatur von 43°C Wachs in der flüssigen Ölkomponente (vgl. Rohstoffe der Tabelle 1 mit der Nummer 1) unter Rühren gelöst.

Die resultierende Wachslösung wurde auf eine Temperatur von höchstens 25°C heruntergekühlt.

Es wurden unter Rühren die Rohstoffe der Nummer 2 gemäß Tabelle 2 zugegeben.

Bei einer Temperatur von 25°C wurde in die Wachslösung die Feststoffe in Partikelform mit der Nummer 3 gemäß Tabelle 1 unter Rühren und zusätzlich unter Verwendung eines Homogenisators für 20 Minuten dispergiert.

Bei einer Temperatur von 25°C wurden die Inhaltsstoffe der Nummer 4 gemäß Tabelle 1 unter Rühren zugegeben.

Bei einer Temperatur von 25°C wurde unter Rühren das Silica (pyrogen) als Verdickungsmittel für die Verdickung von Öl zugegeben.

Anschließend wurde der Gasdruck im Rührgefäß auf einen Überdruck von +150 mbar im Vergleich zum Umgebungsdruck erhöht und die Mischung für einen Zeitraum von 30 Minuten gerührt.

## Patentansprüche

1. Kosmetisches Mittel, enthaltend jeweils bezogen auf das Gesamtgewicht des Mittels
- 0 bis 3 Gew.-%, insbesondere 0 bis 2 Gew.-%, freies Wasser,
- ein oder mehrere flüssige Öle in einer Gesamtmenge von 40 bis 80 Gew.-%, insbesondere von 50 bis 75 Gew.-%, insbesondere von 60 bis 70 Gew.-%, umfassend
∘ mindestens ein Silikonöl
∘ mindestens einen Ester der Formel (I) enthält 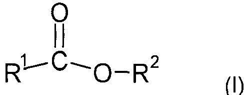 worin R¹ und R² unabhängig voneinander für einen linearen oder verzweigten Kohlenwasserstoffrest mit 3 bis 22 Kohlenstoffatomen stehen
- 0,05 bis 8,0 Gew.-% Wachs mit einem Schmelzpunkt von 30 bis 50°C,
- 10 bis 30 Gew.-% partikelförmig dispergierten Feststoff aus der Gruppe der Metalloxide,
- mindestens ein Verdickungsmittel für die Verdickung von Öl aus der Gruppe der pyrogenen Kieselsäuren,
**dadurch gekennzeichnet, dass**
- das kosmetische Mittel eine Viskosität von 300000 bis 800000 mPas, insbesondere von 400000 bis 600000 mPas (jeweils gemessen bei 20°C, Brookfield, 4 Umdrehungen pro Minute, Spindel TE) besitzt,
- das kosmetische Mittel bei 20°C eine Dichte von 0,95 bis 1,10 g/cm³ hat.

2. Kosmetisches Mittel, nach Anspruch 1, **dadurch gekennzeichnet, dass** es Wachs in einer Gesamtmenge von 0,1 bis 5,0 Gew.-%, besonders bevorzugt von 0,5 bis 3,0 Gew.-%, enthält.

3. Kosmetisches Mittel nach einem der vorangehenden Ansprüche, **dadurch gekennzeichnet, dass** mindestens die Hälfte der Menge des enthaltenen Wachses (bevorzugt mindestens 75% des enthaltenen Wachses) gelöst vorliegt.

4. Kosmetisches Mittel nach einem der vorangehenden Ansprüche, **dadurch gekennzeichnet, dass** es als Wachs mindestens ein Wachs mit einem Schmelzpunkt von 30 bis 40°C, enthält.

5. Kosmetisches Mittel nach einem der vorangehenden Ansprüche, **dadurch gekennzeichnet, dass** es als partikelförmig dispergierten Feststoff mindestens eine Stärke enthält.

6. Kosmetisches Mittel nach Anspruch 1, **dadurch gekennzeichnet, dass** mindestens ein Emulgator an den partikelförmig dispergierten Feststoff adsorbiert, der die Bildung einer Wasser-in-Öl-Emulsion fördert.

7. Kosmetisches Mittel nach Anspruch 6, **dadurch gekennzeichnet, dass** mindestens ein Emulgator an den partikelförmig dispergierten Feststoff adsorbiert, der 2 bis 6 aneinander kovalent bindende Glyceryleinheiten und mindestens eine (insbesondere verzweigte) Alkylgruppe mit 8 bis 20 Kohlenstoffatomen umfasst.

8. Herstellungsverfahren für kosmetische Mittel nach Anspruch 1, worin
- Wachs in einer flüssigen Ölphase unter Bildung einer Wachslösung gelöst wird,
- in diese Wachslösung Feststoff in Partikelform dispergiert wird
- mindestens ein Verdickungsmittel für die Verdickung von Öl zugefügt wird,
- und in die resultierende Mischung oder während des Mischvorganges gasförmiger Stoff dispergiert wird, bis das kosmetische Mittel eine Dichte (gemessen bei 20°C) von 0.95 bis 1.10 g/cm³ besitzt.

9. Verwendung eines kosmetischen Mittels nach einem der Ansprüche 1 bis 7 als Hautkosmetikum.

## Claims

1. A cosmetic agent containing, in each case based on the total weight of the agent:
- 0 to 3 wt.%, in particular 0 to 2 wt.%, free water,
- one or more liquid oils in a total amount of from 40 to 80 wt.%, in particular from 50 to 75 wt.%, in particular from 60 to 70 wt.%, comprising
∘ at least one silicone oil
∘ at least one ester of formula (I) 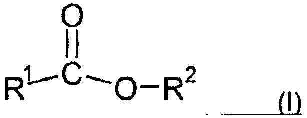 in which R¹ and R² represent, independently of one another, a linear or branched hydrocarbon functional group having 3 to 22 hydrogen atoms
- 0.05 to 8.0 wt.% wax having a melting point of from 30 to 50°C,
- 10 to 30 wt.% solid matter dispersed in particulate form, from the group of metal oxides,
- at least one thickener for thickening oil from the group of pyrogenic silicic acids,
**characterized in that**
- the cosmetic agent has a viscosity of from 300,000 to 800,000 mPa·s, in particular from 400,000 to 600,000 mPas (in each case measured at 20°C, Brookfield, 4 rotations per minute, spindle TE),
- the cosmetic agent has a density of from 0.95 to 1.10 g/cm³ at 20°C.

2. The cosmetic agent according to claim 1, **characterized in that** it contains wax in a total amount of from 0.1 to 5.0 wt.%, particularly preferably from 0.5 to 3.0 wt.%.

3. The cosmetic agent according to one of the preceding claims, **characterized in that** at least half of the amount of contained wax (preferably at least 75% of the contained wax) is dissolved.

4. The cosmetic agent according to one of the preceding claims, **characterized in that** it contains at least one wax having a melting point of from 30 to 40°C as the wax.

5. The cosmetic agent according to one of the preceding claims, **characterized in that** it contains at least one starch as the solid matter dispersed in particulate form.

6. The cosmetic agent according to claim 1, **characterized in that** at least one emulsifier is adsorbed to the solid matter dispersed in particulate form and promotes the formation of a water-in-oil emulsion.

7. The cosmetic agent according to claim 6, **characterized in that** at least one emulsifier is adsorbed to the solid matter dispersed in particulate form and comprises 2 to 6 glycerol units that covalently bond to one another and at least one (in particular branched) alkyl group having 8 to 20 carbon atoms.

8. A method for producing cosmetic agents according to claim 1, in which
- wax is dissolved in a liquid oil phase to form a wax solution,
- solid matter in particulate form is dispersed into this wax solution,
- at least one thickener for thickening oil is added,
- and gaseous material is dispersed into the resulting mixture or during the mixing process until the cosmetic agent has a density (measured at 20°C) of from 0.95 to 1.10 g/cm³.

9. The use of a cosmetic agent according to one of claims 1 to 7 as a skin cosmetic.

## Revendications

1. Produit cosmétique contenant, rapporté respectivement au poids total du produit :
- 0 à 3 % en poids, en particulier 0 à 2 % en poids d'eau libre,
- une ou plusieurs huiles liquides à hauteur totale de 40 à 80 % en poids, en particulier 50 à 75 % en poids, en particulier 60 à 70 % en poids, comprenant :
∘ au moins une huile de silicone
∘ au moins un ester de formule (I) 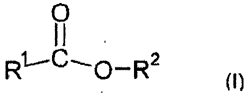 où R¹ et R² représentent, indépendamment l'un de l'autre, un résidu hydrocarbure linéaire ou ramifié avec 3 à 22 atomes de carbone,
- 0,05 à 8,0 % en poids d'une cire avec un point de fusion de 30 à 50°C,
- 10 à 30 % en poids de solide particulaire dispersé du groupe des oxydes métalliques,
- au moins un épaississant pour l'épaississement d'huile, issu du groupe des acides siliciques pyrogènes,
**caractérisé en ce que** :
- le produit cosmétique possède une viscosité de 300 000 à 800 000 mPa.s, en particulier de 400 000 à 600 000 mPa.s (respectivement mesuré à 20°C, Brookfield, 4 tr/min, rotor TE),
- le produit cosmétique a une densité à 20°C de 0,95 à 1,10 g/cm³.

2. Produit cosmétique selon la revendication 1, **caractérisé en ce qu'**il contient une cire à hauteur totale de 0,1 à 5,0 % en poids, particulièrement préférentiellement de 0,5 à 3,0 % en poids.

3. Produit cosmétique selon une des revendications précédentes, **caractérisé en ce qu'**au moins la moitié de la quantité de la cire contenue (de préférence au moins 75 % de la cire contenue) est présente sous forme dissoute.

4. Produit cosmétique selon une des revendications précédentes, **caractérisé en ce qu'**il contient comme cire au moins une cire avec un point de fusion de 30 à 40°C.

5. Produit cosmétique selon une des revendications précédentes, **caractérisé en ce qu'**il contient comme solide particulaire dispersé au moins un amidon.

6. Produit cosmétique selon la revendication 1, **caractérisé en ce qu'**au moins un émulsifiant est adsorbé sur le solide particulaire dispersé, qui favorise la formation d'une émulsion eau dans l'huile.

7. Produit cosmétique selon la revendication 6, **caractérisé en ce qu'**au moins un émulsifiant est adsorbé sur le solide particulaire dispersé, qui comprend 2 à 6 unités glycérine liées les unes aux autres de façon covalente et au moins un groupe alkyle (en particulier ramifié) avec 8 à 20 atomes de carbone.

8. Procédé de fabrication de produit cosmétique selon la revendication 1, dans lequel :
- une cire est dissoute dans une phase huileuse liquide en formant une solution de cire,
- un solide est dispersé sous forme particulaire dans cette solution de cire,
- au moins un épaississant est ajouté pour épaissir l'huile,
- et dans le mélange obtenu ou pendant le processus de mélange, une substance gazeuse est dispersée jusqu'à ce que le produit cosmétique possède une densité (mesurée à 20°C) de 0,95 à 1,10 g/cm³.

9. Utilisation d'un produit cosmétique selon une des revendications 1 à 7 comme cosmétique pour la peau.
